# DEMANDE DE BREVET EUROPEEN

(11) **EP 0 897 704 A1**
(43) Date de publication de la demande: **24.02.1999**
(21) Numéro de dépôt: 98490020.9
(22) Date de dépôt: 17.07.1998
(51) Int. Cl.: A61F 2/44

(54) **Implant pour établir une fusion intervertébrale**

(30) Priorité: 18.07.1997 FR 9709443
(71) Demandeur: Carlier, Hervé, 59113 Seclin (FR); Cartoux, René, 84220 Gordes (FR)
(72) Inventeur: Carlier, Hervé, 59113 Seclin (FR); Cartoux, René, 84220 Gordes (FR)
(74) Mandataire: Ecrepont, Robert

(57) **Abrégé**

L'invention se rapporte à un implant pour établir une fusion intervertébrale comprenant :
- une enveloppe (2) cylindrique présentant une cavité (3) axiale pour y loger un greffon osseux,
- un bouchon de fermeture pour obturer l'extrémité ouverte,
- des moyens (7) de maintien provisoire de l'implant entre les structures osseuses et cette enveloppe, dans les zones situées en regard des structures osseuses à relier par un pont de matière osseuse, présente de larges lumières (8) au travers desquelles la substance osseuse va croître.

## Description

L'invention se rapporte à un implant pour établir une fusion intervertébrale.

Dans certaines pathologies, il est nécessaire de réaliser l'immobilisation de deux corps vertébraux pour éviter les déplacements de l'un par rapport à l'autre.

Si cette immobilisation peut être réalisée à l'aide de plaques maintenues en place par des vis, broches ou autres, il est connu d'obtenir cette immobilisation par création d'un pontage osseux entre les deux corps vertébraux à immobiliser.

A cet effet, dans la zone où ce pontage doit être réalisé, on implante entre les corps vertébraux à associer un greffon osseux, lequel va développer avec les structures osseuse adjacentes de la substance osseuse qui, à terme, formera la liaison entre lesdites vertèbres.

Pour ce faire, au lieu d'introduire directement le greffon dans une cavité aménagée entre les vertèbres, on place ce greffon dans une pièce ou implant creux, notamment métallique, ou en un autre type de matériau et c'est cet implant qui est introduit dans la cavité précitée.

Cet implant comprend :
- une enveloppe cylindrique de révolution présentant une cavité axiale pour y loger un greffon osseux, laquelle enveloppe a l'une de ses extrémités ouverte pour y insérer le greffon,
- un bouchon de fermeture pour obturer l'extrémité ouverte précitée,
- des moyens de maintien provisoire de l'implant entre les structures osseuses et cette enveloppe, dans les zones situées en regard des structures osseuses à relier par un pont de matière osseuse, présente de larges lumières au travers desquelles la substance osseuse va croître.

On connaît un implant WO-A-96.08205, FR-A-1.228.362 du type précité dans lequel les lumières sont d'étendues réduites, ce qui réduit considérablement la qualité de la liaison osseuse.

La taille réduite de ces lumières permet d'équiper la face externe d'un filet de vis pour la mise en place de l'implant et son maintien provisoire en position.

Les lumières sont ainsi réalisées dans la partie creuse du filetage.

On connaît d'autres implants WO-9531947 ou FR-A-2708461 présentant des lumières en nombre réduit, mais plus étendues.

Compte tenu de l'étendue de ces lumières, il n'est plus fait appel à un filet de vis, mais à de simples nervures.

Ces derniers implants ne peuvent être introduits que par un déplacement selon l'axe longitudinal dudit implant notamment en raison des nervures s'opposant à la rotation.

La présence de larges lumières ne gène pas la mise en place de l'implant dans la mesure où cette mise en place se fait par simple translation.

Un des résultats que l'invention vise à obtenir est un implant du type précité qui, notamment, améliore la vitesse de prise du greffon.

A cet effet, l'invention a pour objet un implant du type précité comprenant :
- une enveloppe cylindrique de révolution présentant une cavité axiale pour y loger un greffon osseux, laquelle enveloppe a l'une de ses extrémités qui est ouverte pour y insérer le greffon,
- un bouchon de fermeture pour obturer l'extrémité ouverte précitée,
- des moyens de maintien provisoire de l'implant entre les structures osseuses et cette enveloppe, dans les zones dites supérieure et inférieure situées en regard des structures osseuses à relier par un pont de matière osseuse, présente de larges lumières au travers desquelles la substance osseuse va croître,
cet implant étant CARACTERISE en ce que les larges lumières ont une forme géométrique de parallélogramme dont deux champs des bords longitudinaux de ces lumières sont parallèles à l'axe longitudinal de l'implant et s'étendent dans des plans délimitant un angle au moins égal à celui défini par les plans radiaux passant par les arêtes dites arêtes internes formées par l'intersection de la surface interne l'enveloppe et des champs précités.

L'invention sera bien comprise à l'aide de la description ci-après faite, à titre d'exemple non limitatif, en regard du dessin annexé qui représente schématiquement :
- figure 1 : un implant vu de face,
- figure 2 : une coupe selon II-II de la figure 1,
- figure 3 : un détail de l'implant,
- figure 4 : coupe d'une variante de réalisation.

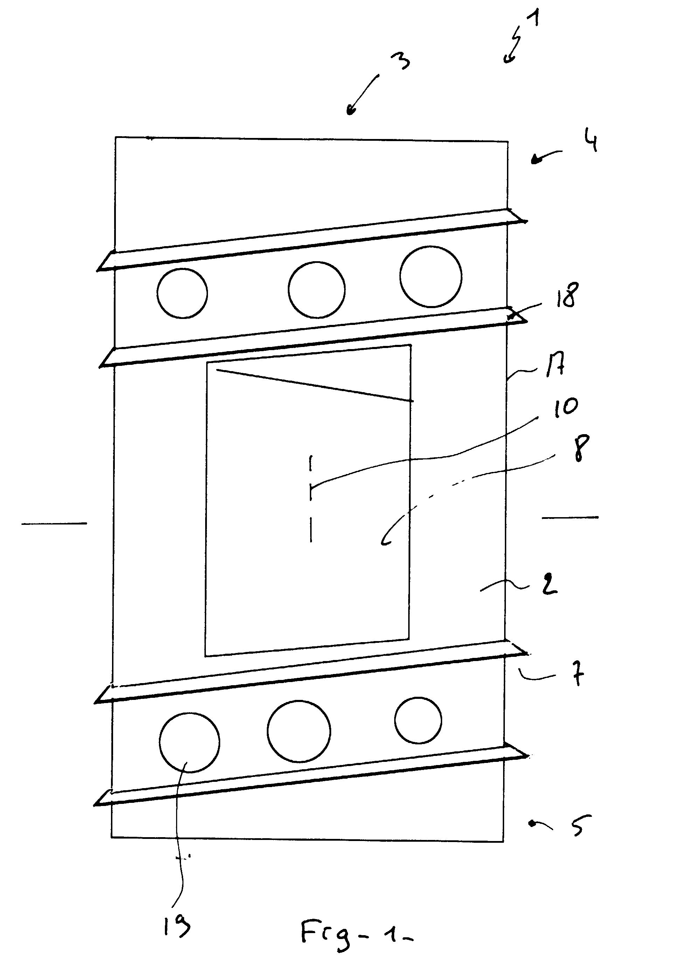

En se reportant au dessin, on voit que l'implant 1 destiné à établir, par croissance du tissus osseux, une fusion entre deux structures osseuses voisines, comprend :
- une enveloppe 2 cylindrique de révolution présentant une cavité 3 axiale pour y loger un greffon osseux, (non représenté) laquelle enveloppe 2 a l'une (4) de ses extrémités 4,5 qui est ouverte pour y insérer le greffon,
- un bouchon 6 de fermeture pour obturer l'extrémité ouverte précitée,
- des moyens 7 de maintien provisoire de l'implant entre les structures osseuses et cette enveloppe, dans les zones situées en regard des structures osseuses à relier par un pont de matière osseuse, présente de larges lumières 8 au travers desquelles la substance osseuse va croître.

Selon une caractéristique de l'invention, les larges lumières 8 ont une forme géométrique de parallélogramme dont deux champs 9 des bords longitudinaux de ces lumières sont parallèles à l'axe 10 longitudinal de l'implant et s'étendent dans des plans 11,12 délimitant un angle A au moins égal à celui B défini par les plans 13,14 radiaux passant par les arêtes dites arêtes 15,16 internes formées par l'intersection de la surface interne de l'enveloppe et des champs précités.

L'angle A défini par les plans radiaux est de préférence au moins égal à 70°(soixante dix degrés).

L'orientation de ces champs 9 facilite la mise en place de l'implant par rotation de cet implant autour de son axe longitudinal.

En effet, ces champs 9 agissent comme des plans inclinés.

Dans l'exemple représenté, ces champs s'étendent dans des plans radiaux.

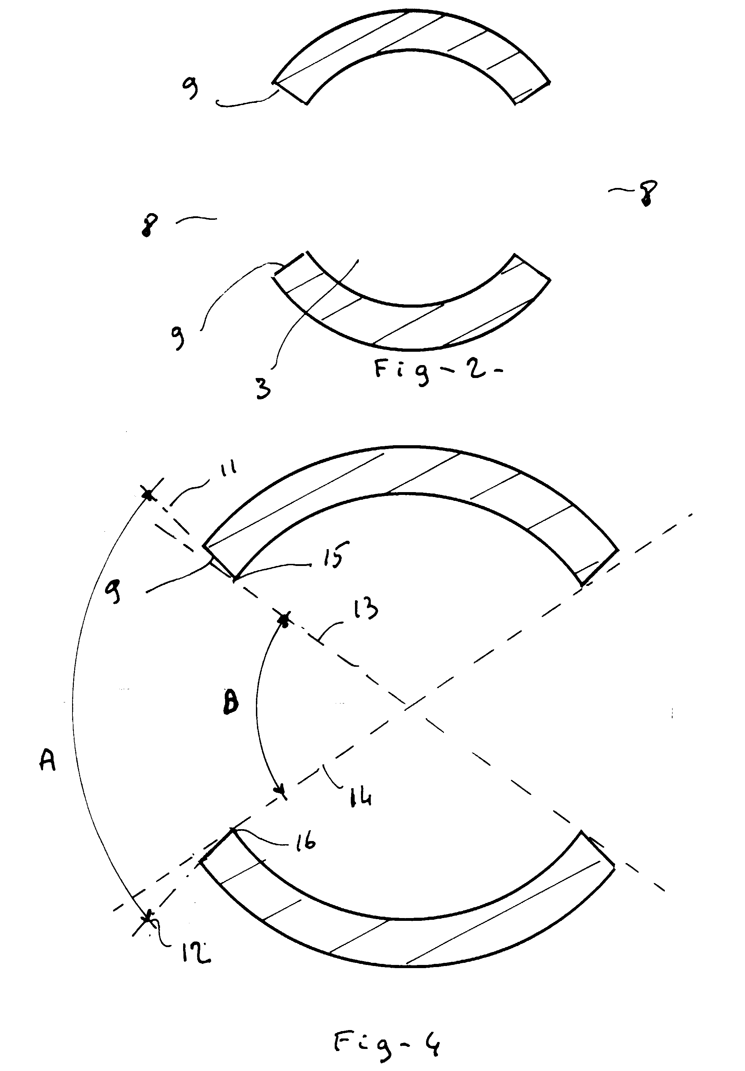

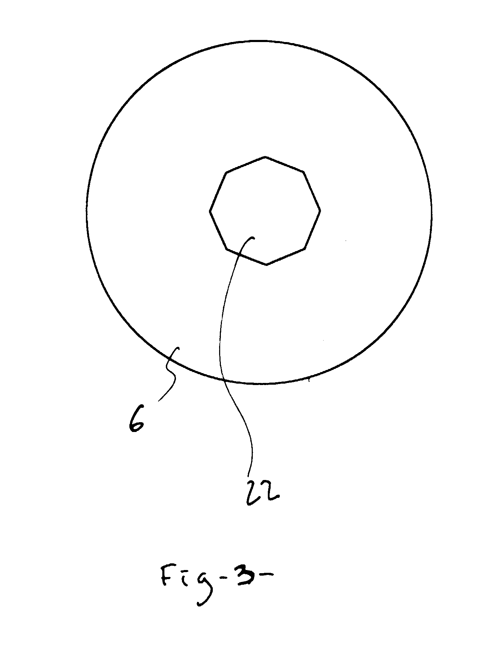

Dans une variante de réalisation (figure 4) l'angle A formé par ces champs est plus ouvert que celui B passant par les arêtes internes.

Un angle A de 90° (quatre vingt dix degrés) est avantageux.

La face 17 externe de l'implant sera équipée localement d'un filet 18 de vis hélicoïdal, de préférence, ayant un profil auto-taraudant.

Au niveau des extrémités de l'implant et dans la zone de l'enveloppe située dans le creux du filetage, cet implant présente des petites lumières 19 supplémentaires.

Le bouchon de fermeture, en titane, est vissé sur l'enveloppe et ce bouchon et le fond de la cavité logeant le greffon sont pourvus d'une perforation 22 axiale permettant une visualisation de la fusion lors d'un contrôle radiographique par scanner.

La forme évasée vers l'extérieur des lumières de l'implant améliore notablement la solidité du pontage osseux.

## Revendications

1. Implant pour établir une fusion intervertébrale comprenant :
- une enveloppe (2) cylindrique de révolution présentant une cavité (3) axiale pour y loger un greffon osseux, (non représenté) laquelle enveloppe (2) a l'une (4) de ses extrémités (4,5) qui est ouverte pour y insérer le greffon,
- un bouchon (6) de fermeture pour obturer l'extrémité ouverte précitée,
- des moyens (7) de maintien provisoire de l'implant entre les structures osseuses et cette enveloppe, dans les zones situées en regard des structures osseuses à relier par un pont de matière osseuse, présente de larges lumières (8) au travers desquelles la substance osseuse va croître
cet implant étant **caractérisé** en ce que les larges lumières (8) ont une forme géométrique de parallélogramme dont deux champs (9) des bords longitudinaux de ces lumières sont parallèles à l'axe (10) longitudinal de l'implant et s'étendent dans des plans (11,12) délimitant un angle (A) au moins égal à celui (B) défini par les plans (13,14) radiaux passant par les arêtes dites arêtes (15,16) internes formées par l'intersection de la surface interne de l'enveloppe et des champs précités.

2. Implant selon la revendication 1 **caractérisé** en ce que l'angle (A) défini par les plans radiaux est de préférence au moins égal à 70°(soixante dix degrés).

3. Implant selon la revendication 1 ou 2 **caractérisé** en ce que l'angle (A) formé par ces champs est plus ouvert que celui (B) passant par les arêtes internes.

4. Implant selon la revendication 3 **caractérisé** en ce qu'un angle (A) de 90° (quatre vingt dix degrés) est avantageux.

5. Implant selon la revendication 1 **caractérisé** en ce que la face (17) externe de l'implant sera équipée localement d'un filet (18) de vis hélicoïdal, de préférence, ayant un profil auto-taraudant.

6. Implant selon la revendication 5 **caractérisé** en ce que au niveau des extrémités de l'implant et dans la zone de l'enveloppe située dans le creux du filetage, cet implant présente des petites lumières (19) supplémentaires.

7. Implant selon la revendication 1 **caractérisé** en ce que le bouchon de fermeture, en titane, est vissé sur l'enveloppe et ce bouchon et le fond de la cavité logeant le greffon sont pourvus d'une perforation (22) axiale permettant une visualisation de la fusion lors d'un contrôle radiographique par scanner.
